(19) Europäisches Patentamt
European Patent Office
Office européen des brevets

(11) Veröffentlichungsnummer: **0 105 029**
**A2**

(12) # EUROPÄISCHE PATENTANMELDUNG

(21) Anmeldenummer: **83810375.2**

(22) Anmeldetag: **22.08.83**

(51) Int. Cl.³: **C 07 D 239/62**
**A 61 K 31/515**

(30) Priorität: **26.08.82 CH 5080/82**

(43) Veröffentlichungstag der Anmeldung:
**04.04.84 Patentblatt 84/14**

(84) Benannte Vertragsstaaten:
**BE CH DE FR GB IT LI NL SE**

(71) Anmelder: **CIBA-GEIGY AG**
**Postfach**
**CH-4002 Basel(CH)**

(72) Erfinder: **De Sousa, Bernardo**
**Paradiesstrasse 15**
**CH-4125 Riehen(CH)**

(72) Erfinder: **Gallay, Jean Jacques, Dr.**
**Blumenrain 19**
**CH-4312 Magden(CH)**

(54) **Barbitursäurederivate als Anthelmintika.**

(57) Anthelmintische Mittel, die als aktive Komponente Phenylbarbitursäurederivate der Formel

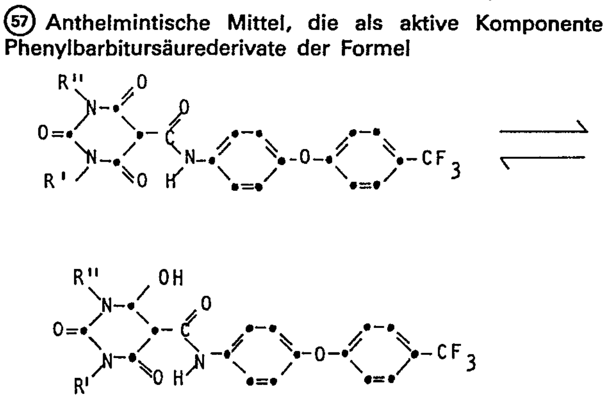

in welcher R' und R" unabhängig voneinander Wasserstoff oder $C_1$-$C_5$-Alkyl bedeuten, einschliesslich ihrer tautomeren Formen und ihrer Salze neben Trägerstoffen und weiteren Formulierungshilfsstoffen enthalten. Die Mittel dienen zur Bekämpfung von tierparasitären Helminthen.

EP 0 105 029 A2

CIBA-GEIGY AG
Basel (Schweiz)

5-14068/B

BEZEICHNUNG GEÄNDERT
siehe Titelseite

Anthelmintische Mittel

Die vorliegende Erfindung betrifft anthelmintische Mittel enthaltend als aktive Komponente 5-Phenylcarbamoylbarbitursäurederivate sowie ihre Verwendung zur Bekämpfung von Nematoden, Cestoden und Trematoden in Haus- und Nutztieren.

Die Erfindung betrifft ferner die Herstellung der anthelmintischen Mittel.

Die in den erfindungsgemässen Mitteln als Aktivsubstanzen verwendeten Verbindungen entsprechen der allgemeinen Formel I

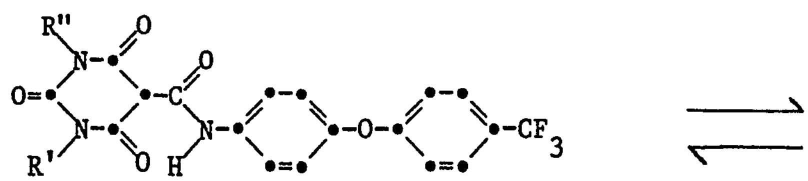

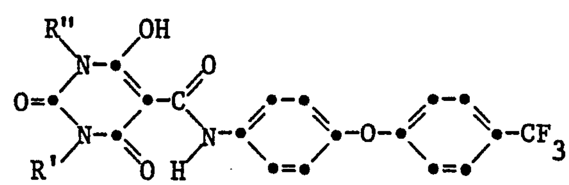

(I)

in welcher R' und R" unabhängig voneinander Wasserstoff oder $C_1$-$C_5$-Alkyl bedeuten, einschliesslich ihrer tautomeren Formen und ihrer Salze.

Zu den betreffenden Salzen zählen beispielsweise insbesondere die Alkalimetall-, Ammonium- oder Aminsalze, wobei Natrium-, Kalium-, Ammonium- oder Alkylaminsalze, besonders Triäthylaminsalze, bevorzugt sind.

Gemäss Formel I sind unter Alkylgruppen sowohl gerade als auch verzweigtkettige zu verstehen. Dazu zählen die Methyl-, die Aethyl- sowie die Isomeren der Propyl-, der Butyl- und der Pentylgruppe.

Als bevorzugt sind Verbindungen der Formel I anzusehen, in denen R' oder R" unabhängig voneinander Methyl, Aethyl, n-Propyl, iso-Propyl oder iso-Pentyl bedeutet.

Die unter die Formel I fallenden Verbindungen sind in der Europäischen Patentanmeldung Nr. 7,541 in allgemeiner Form beschrieben.

Dagegen sind sowohl der Umfang der Verbindungen gemäss Formel I als auch die unter die Formel I fallenden Einzelverbindungen neu und stellen einen Teil der Erfindung dar.

Nach Angaben der Europäischen Patentanmeldung werden den darin genannten Verbindungen insektizide, akarizide und fungizide Eigenschaften zugeschrieben.

Es wurde nun überraschenderweise und unvorhersehbar gefunden, dass die Verbindungen der Formel I und die sie als Aktivsubstanzen enthaltenden Mittel ein breites Wirkungsspektrum gegen im Tierorganismus, vor allem in Säugetieren, parasitierende Helminthen besitzen. Sie sind gleichermassen gegen Nematoden, Cestoden und Trematoden mit gutem Erfolg anwendbar.

Die neuen Wirkstoffe der Formel I werden hergestellt, indem man entweder

a) einen Ester der Formel II

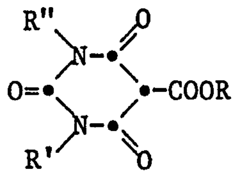

(II)

mit einem Anilinderivat der Formel III reagieren lässt,

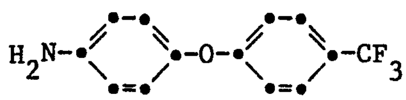

(III)

worin R eine Alkyl- oder eine gegebenenfalls substituierte Phenylgruppe darstellt und die Reste R' und R" die unter der Formel I angegebenen Bedeutungen besitzen, oder

b) eine substituierte Barbitursäure der Formel IV

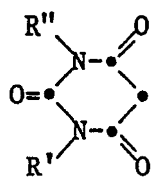

(IV)

mit einem substituierten Phenylisocyanat der Formel V umsetzt,

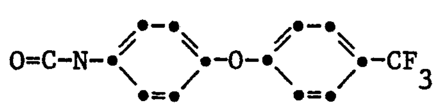

(V)

worin die Reste R' und R" die unter Formel I angegebenen Bedeutungen besitzen,
oder

c) eine substituierte Barbitursäure der Formel IV mit einem substituierten Benzoylazid der Formel VI reagieren lässt,

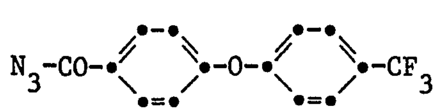

(VI),

worin die Reste R' und R" die unter Formel I angegebenen Bedeutungen besitzen.

Die Herstellungsverfahrensvarianten (a) und (c) werden im allgemeinen bei Reaktionstemperaturen zwischen 80° und 250°C, vorzugsweise 100° bis 220°C, durchgeführt. Variante (b) erfordert Reaktionstemperaturen zwischen 0° und 220°C, insbesondere 0° und 200°C. Die Reaktionen (a), (b) und (c) können bei normalem oder erhähtem Druck und in Abwesenheit oder vorzugsweise in Gegenwart reaktionsinerter Lösungs- oder Verdünnungsmittel durchgeführt werden, wobei in manchen Fällen vorteilhafterweise mit einer Base gearbeitet wird.

Die Herstellung der Salze von Verbindungen der Formel I erfolgt durch übliche Neutralisation der freien Säure mit einer Base, insbesondere einer physiologisch verträglichen Base. Vorzugsweise genannt seien Alkalisalze wie Natrium-, Kalium- oder Lithiumsalze sowie Ammoniumsalze und Trialkylaminsalze wie z.B. das bevorzugte Triethylaminsalz. Die Neutralisation wird in einem reaktionsinerten polaren Lösungsmittel, z.B. einem Alkanol, Ester oder einer etherartigen Verbindung, durchgeführt.

Für die Herstellung der Wirksubstanzen geeignete Lösungs- oder Verdünnungsmittel sind z.B. Ether und etherartige Verbindungen wie Dialkylether (Diethylether, Diisopropylether, tert.-Butylmethylether usw.), Anisol, Dioxan, Tetrahydrofuran; aliphatische und aromatische Kohlenwasserstoffe wie Benzol, Toluol, Petrolether; halogenierte Kohlenwasserstoffe wie Chlorbenzol, Methylenchlorid, Chloroform, Ethylenchlorid, Tetrachlorkohlenstoff, Tetrachlorethylen; Nitrile wie Acetonitril, Propionitril; N,N-dialkylierte Amide wie Dimethylformamid; Dimethylsulfoxid; Ketone wie Aceton, Diethylketon und Gemische solcher Lösungsmittel untereinander.

Als Basen kommen organische und anorganische Basen in Betracht; z.B. vorzugsweise tertiäre Amine wie Trialkylamine (Trimethylamin, Triethylamin, Tripropylamin usw.), Pyridin und Pyridinbasen (z.B. 4-Dimethylaminopyridin, 4-Pyrrolidylaminopyridin usw.), Picoline und Lutidine sowie Oxide, Hydroxide, Carbonate und Hydrogencarbonate von Alkali- und Erdalkalimetallen (z.B. CaO, BaO, NaOH, KOH, $Ca(OH)_2$, $KHCO_3$, $NaHCO_3$, $Ca(HCO_3)$, $K_2CO_3$, $Na_2CO_3$ usw.), ferner Acetate wie z.B. $CH_3COONa$ oder $CH_3COOK$. Darüber hinaus eignen sich als Basen auch Alkalialkoholate wie z.B. Natriummethylat, Natriumpropylat, Kalium-tert.-butylat oder Natriumethylat. Die Base wird in bezug auf die Reaktanden in 10 bis 100% der äquimolaren Menge zugesetzt.

In manchen Fällen kann es von Vorteil sein, wenn die Reaktion unter Schutzgasatmosphäre durchgeführt wird. Geeignete Schutzgase sind z.B. Stickstoff, Helium, Argon oder Kohlendioxid.

Die in den Herstellungsvarianten (a), (b) und (c) genannten Ausgangsstoffe sind bekannt (siehe z.B. Chem. Ber. 54, 1038 [1921]) oder können analog den bekannten Substanzen hergestellt werden.

Die Wirkstoffe der Formel I können in unterschiedlichen tautomeren Formen vorliegen, nämlich in der Keto- oder Enolform oder in einem Gemisch aus Keto- und Enolform. Als Wirkstoffe der erfindungsgemässen Mittel sind demzufolge sowohl die einzelnen Tautomeren als auch deren Gemische, aber auch die Salze jeder dieser Formen anzusehen.

In den erfindungsgemässen Verfahren zur Bekämpfung von parasitären Helminthen werden die Wirkstoffe der Formel I sowohl als Aktivkomponenten in den erfindungsgemässen Mitteln als auch allein eingesetzt.

Die Erfindung betrifft auch ein Verfahren zum Schutz von Tieren vor parasitären Helminthen, das dadurch gekennzeichnet ist, dass man die Wirkstoffe der Formel I bzw. die Wirkstoffformulierungen als Zusatz zum Futter oder zu den Tränken oder auch in fester oder flüssiger Form oral, durch Injektion oder mittels der Pour-on-Methode den Tieren appliziert.

Unter den bei Warmblütern vorkommenden Endoparasiten verursachen namentlich die Helminthen grosse Schäden. So zeigen zum Beispiel von diesen Parasiten befallene Tiere nicht nur ein verlangsamtes Wachstum, sondern teilweise sogar Schädigungen, die zu ihrem Absterben führen können. Daher ist es von grosser Bedeutung, therapeutische Mittel zu entwickeln, die sich zur Bekämpfung von Helminthen und deren Entwicklungsstadien, sowie zur Vorbeugung gegen den Befall durch diese Parasiten eignen. Besonders gefährtliche Wurmkrankheiten sind solche, die durch im Magen-Darmtrakt und anderen Organen parasitierende Nematoden, Cestoden und Trematoden hervorgerufen werden und vor allem bei Wiederkäuern, wie Schafen, Rindern und Ziegen, sowie Equiden und Geflügel auftreten.

Die durch Helminthiasen verursachten Schäden können bei chronischem und vor allem bei epidemischem Auftreten der Infestation in Viehherden beträchtlich sein. Sie äussern sich unter anderem in Produktivitäts-Verminderungen, geschwächter Widerstandskraft und erhöhter Mortalität. Bekämpfung und Vorbeugung von Helminthiasen gelten deshalb als vordringliche Aufgabe, um derartige, insbesondere volkswirtschaftlich ins Gewicht fallende, Schäden zu vermeiden oder zu mindern.

In der vorliegenden Beschreibung werden unter dem Begriff "Helminthen" insbesondere solche parasitären Würmer verstanden, die zu den Phyla Platyhelminthes (Cestoden, Trematoden) und Nemathelminthes (Nematoden und Verwandte) gehören, also Bandwürmer, Saugwürmer und Rundwürmer des Gastrointestinal-Traktes und anderer Organe (z.B. Leber, Lunge, Niere, Lymphgefässe, Blut etc.). Es sind zwar eine Reihe von Stoffen

mit anthelmintischer Wirkung bekannt, die für die Bekämpfung der verschiedenen Helminthen species vorgeschlagen wurden. Diese vermögen jedoch nicht voll zu befriedigen, sei es, dass bei verträglicher Dosierung eine Ausschöpfung ihres Wirkungsspektrums nicht möglich ist, oder dass sie in therapeutisch wirksamen Dosen unerwünschte Nebenwirkungen oder Eigenschaften zeigen. In diesem Zusammenhang spielt auch die heute vermehrt auftretende Resistenz gegen bestimmte Stoffklassen eine immer bedeutendere Rolle. Das beispielsweise in der Literatur beschriebene Albendazol (British Pat. No. 1464326; Am. J. Vet. Res. 38, 1425-1426 (1977); Am. J. Vet. Res. 37, 1515-1516 (1976); Am J. Vet. Res. 38, 807-808 (1977); Am. J. Vet. Res. 38, 1247-1248 (1977)) besitzt als bekanntes Anthelmintikum zwar ein Wirkungsspektrum bei Wiederkäuern, jedoch ist z.B. seine Wirkung gegen Benzimidazol-resistente Nematoden und adulte Leberegel völlig unzureichend, wobei vor allem die pathologisch wichtigen unreifen Wanderformen der letzteren bei den verträglichen Dosierungen nicht angegriffen werden.

Ueberraschenderweise wurde nun festgestellt, dass die Wirkstoffe der Formel I und die sie als Aktivkomponente enthaltenden erfindungsgemässen Mittel sowohl eine intensive anthelmintische Wirksamkeit mit breitem Wirkungsspektrum gegen Nematoden, Cestoden und Trematoden als auch zusätzlich eine günstige Warmblütertoxizität besitzen.

Die Wirkstoffe der Formel I und die sie enthaltenden erfindungsgemässen Mittel sind beispielsweise zur Bekämpfung parasitärer Nematoden der Ordnungen (nach K.I. Skrajabin)

Rhabditida
Ascaridida
Spirurida
Trichocephalida

oder zur Bekämpfung von Cestoden der Ordnungen (nach Wardle & McLeod)

Cyclophyllidea

Pseudophyllidea

oder zur Bekämpfung von Trematoden der Ordnung

Digenea

bei Haus- und Nutztieren, wie Rindern, Schafen, Ziegen, Pferden, Schweinen, Katzen, Hunden und Geflügel, geeignet. Sie können den Tieren sowohl als Einzeldosis als auch wiederholt verabreicht werden, wobei die einzelnen Gaben je nach Tierart vorzugsweise zwischen 1 und 100 mg pro kg Körpergewicht betragen. Durch eine protrahierte Verabreichung erzielt man in manchen Fällen eine bessere Wirkung oder man kann mit geringeren Gesamtdosen auskommen.

Die erfindungsgemässen Mittel werden hergestellt, indem die Wirkstoffe der Formel I mit flüssigen und/oder festen Formulierungshilfsstoffen durch schrittweises Vermischen und/oder Vermahlen derart in Kontakt gebracht werden, dass eine anwendungskonforme optimale Entfaltung der anthelmintischen Aktivität der Formulierung erzielt wird.

Die Formulierungsschritte können durch Kneten, Granulieren (Granulate) und gegebenenfalls Pressen (Pellets) ergänzt werden.

Als Formulierungshilfsstoffe dienen beispielsweise feste Trägerstoffe, Lösungsmittel und gegebenenfalls oberflächenaktive Stoffe (Tenside).

Zur Bereitung der erfindungsgemässen Mittel werden folgende Formulierungshilfsstoffe verwendet:

Feste Trägerstoffe wie z.B. Kaolin, Talkum, Bentonit, Kochsalz, Calciumphosphat, Kohlenhydrate, Cellulosepulver, Baumwollsaatmehl, Polyäthylenglykoläther, gegebenenfalls Bindemittel wie z.B. Gelatine, lösliche Cellulosederivate, gewünschtenfalls unter Zusatz von oberflächenaktiven Stoffen wie ionischen oder nicht-ionischen Dispersionsmitteln; ferner natürliche Gesteinsmehle wie Calcit,

Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinertes Pflanzenmaterial verwendet werden.

Als Lösungsmittel kommen in Frage: Aromatische Kohlenwasserstoffe, bevorzugt die Franktionen $C_8$ bis $C_{12}$, wie z.B. Xylolgemische oder substituierte Naphthaline, Phthalsäureester wie Dibutyl- oder Dioctylphthalat; aliphatische Kohlenwasserstoffe wie z.B. Cyclohexan oder Paraffine, Alkohole und Glykole sowie deren Ether und Ester, wie z.B. Ethanol, Ethylenglykol, Ethylenglykolmonomethyl- oder Ethylether, Ketone wie z.B. Cyclohexanon, stark polare Lösungsmittel wie z.B. N-Methyl-2-pyrrolidon, Dimethylsulfoxid oder Dimethylformamid, sowie gegebenenfalls epoxydierte Pflanzenöle wie z.B. epoxydiertes Kokosnussöl oder Sojaöl und Wasser.

Als oberflächenaktive Verbindungen kommen je nach Art des zu formulierenden Wirkstoffes der Formel I nicht-ionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein. Auch Phospholipide wie Lecithin lassen sich einsetzen.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen

Fettsäuregemischen, die z.B. aus Kokosnuss- oder Talgöl gewonnen werden können. Ferner sind auch die Fettsäure-methyllaurinsalze zu erwähnen.

Häufig werden sog. synthetische Tenside verwendet, insbesondere Fettsulfonate, Fettsulfate, sulfonierte Benzimidazolderivate oder Alkylsulfonate.

Ferner kommen auch entsprechende Phosphate wie z.B. Salze des Phosphorsäureesters eines p-Nonylphenol-(4-14)-Ethylenoxyd-Adduktes in Frage.

Als nicht-ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Ferner kommen auch Fettsäureester von Polyoxyethylensorbitan wie das Polyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die in der Formulierungstechnik gebräuchlichen Tenside sind u.a. in folgenden Publikationen beschrieben:

"Mc Cutcheon's Detergents and Emulsifiers Annual" BC Publishing Corp., Ringwood New Jersey, 1980;
Sisely and Wood, "Encyclopedia of Surgace Active Agents", Chemical Publishing Co., Inc. New York, 1980.

Als Bindemittel für Tabletten und Boli kommen chemisch abgewandelte, in Wasser oder Alkohol lösliche, polymere Naturstoffe in Frage, wie Stärke-, Cellulose- oder Protein-derivate (z.B. Methylcellulose, Carboxymethylcellulose, Ethylhydroxyethylcellulose, Proteine wie Zein, Gelatine und dergleichen) sowie synthetische Polymere wie z.B. Polyvinylalkohol, Polyvinylpyrrolidon etc.. Ferner sind in Tabletten Füllstoffe, (z.B. Stärke, mikrokristalline Cellulose, Zucker, Milchzucker etc.), Gleitmittel und Sprengmittel enthalten.

Liegen die anthelmintischen Mittel in Form von Futterkonzentraten vor, so dienen als Trägerstoffe zum Beispiel Leistungsfutter, Futtergetreide oder Proteinkonzentrate. Solche Futterkonzentrate oder -mittel können ausser den Wirkstoffen noch Zusatzstoffe, Vitamine, Antibiotika, Chemotherapeutika, oder andere Pestizide, vornehmlich Bakteriostatika, Fungistatika, Coccidiostatika, oder auch Hormonpräparate, Stoffe mit anaboler Wirkung oder andere das Wachstum begünstigende, die Fleischqualität von Schlachttieren beeinflussende oder in anderer Weise für den Organismus nützliche Stoffe enthalten. Werden die Mittel oder die darin enthaltenen Wirkstoffe der Formel I direkt dem Futter oder den Viehtränken zugesetzt, so enthält das Fertigfutter oder die Fertigtränke die Wirkstoffe vorzugsweise in einer Konzentration von etwa 0,0005 bis 0,02 Gewichtsprozent (5-200 ppm).

Die Applikation der erfindungsgemässen Mittel an die zu behandelnden Tiere kann peroral, parenteral, subcutan oder topikal durchgeführt werden, wobei die Mittel in Form von Lösungen, Emulsionen, Suspensionen (Drenchs), Pulvern, Tabletten, Bolussen und Kapseln vorliegen.

Die erfindungsgemässen anthelmintischen Mittel enthalten in der Regel 0,1 bis 99 Gew.-%, insbesondere 0,1 bis 95 Gew.-% Wirkstoff der Formel I, 99,9 bis 1 Gew.-%, insbesondere 99,8 bis 5 Gew.-%, eines festen oder flüssigen Zusatzstoffes, darunter 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Solche Mittel können noch weitere Zusätze wie Stabilisatoren, Entschäumer, Viskositätsregulatoren, Bindemittel sowie Haftmittel oder andere Wirkstoffe zur Erzielung spezieller Effekte enthalten.

Derartige vom Endverbraucher verwendete anthelmintische Mittel sind ebenfalls ein Bestandteil der vorliegenden Erfindung.

Herstellungbeispiel:

Beispiel 1: Herstellung von 1,3-Dimethyl-5-[4-(4-trifluormethylphenoxy)-phenylcarbamoyl]-barbitursäure

45,7 g (0,2 Mol) 1,3-Dimethyl-5-ethoxycarbonyl-barbitursäure und 50,4 g (0,2 Mol) 4-(4-Trifluormethylphenoxy)-anilin werden in 500 ml Toluol suspendiert und unter einem Schutzgasstrom (Stickstoff) 14 Stunden auf Rückflusstemperatur erhitzt, wobei Ethanol entweicht.

Anschliessend wird das Gemisch auf Raumtemperatur abgekühlt und durch Absaugen filtriert. Das abfiltrierte Produkt wird mit Ethanol nachgewaschen und anschliessend getrocknet.

Ausbeute: 81,4 g (= 93,5% d.Th.) Smp. 192-194°C.

Tabelle I

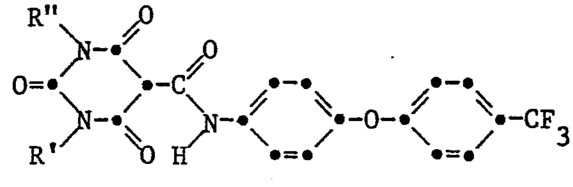

| Nr. | R' | R" | Schmelzpunkt in °C |
| --- | --- | --- | --- |
| 1 | $-CH_3$ | $-CH_3$ | 192-194 |
| 2 | $-CH_3$ | H | 245-250 |
| 3 | $-CH_3$ | $-C_2H_5$ | 181-184 |
| 4 | $-CH_3$ | $-i-C_3H_7$ | 178-180 |
| 5 | $-CH_3$ | $-i-C_4H_9$ | |
| 6 | $-CH_3$ | $-n-C_3H_7$ | |
| 7 | $-C_2H_5$ | $-C_2H_5$ | 186-188 |
| 8 | $-i-C_3H_7$ | $-i-C_3H_7$ | |

Beispiele für Formulierungen

(% = Gewichtsprozent)

| 2. Emulsions-Konzentrate | a) | b) | c) |
| --- | --- | --- | --- |
| Wirkstoff aus Tabelle I | 25% | 40% | 50% |
| Ca-Dodecylbenzolsulfonat | 5% | 8% | 6% |
| Ricinusöl-polyethylenglykolether (36 Mol Ethylenoxid) | 5% | - | - |
| Tributylphenol-polyethylenglycolether (30 Mol Ethylenoxid) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden.

| 3. Granulate | a) | b) |
|---|---|---|
| Wirkstoffe aus Tabelle I | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum verdampft.

| 4. In Wasser dispergierbare Pulvermischung | a) | b) | c) |
|---|---|---|---|
| Wirkstoff aus Tabelle I | 25% | 50% | 75% |
| Na-Ligninsulfonat | 5% | 5% | - |
| Oelsäure | 3% | - | 5% |
| Na-Diisobutylnaphthalinsulfonat | - | 6% | 10% |
| Octylphenolpolyethylenglykolether (7-8 Mol Ethylenoxid) | - | 2% | - |
| Hochdisperse Kieselsäure | 5% | 10% | 10% |
| Kaolin | 62% | 27% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 5. Emulsions-Konzentrat | a) | b) | c) |
|---|---|---|---|
| Wirkstoff aus Tabelle I | 10% | 8% | 60% |
| Octylphenolpolyethylenglykolether (4-5 Mol Ethylenoxid) | 3% | 3% | 2% |
| Ca-Dodecylbenzolsulfonat | 3% | 4% | 4% |
| Ricinusölpolyglykolether (35 Mol Ethylenoxid) | 4% | 5% | 4% |
| Cyclohexanon | 30% | 40% | 15% |
| Xylolgemisch | 50% | 40% | 15% |

Aus diesem Konzentrat können durch Verdünnen mit Wasser Emulsionen jeder gewünschten Konzentration hergestellt werden.

6. Suspension-Konzentrat

| | |
|---|---|
| Wirkstoff aus Tabelle I | 40% |
| Ethylenglykol | 10% |
| Nonylphenolpolyethylenglykolether (15 Mol Ethylenoxid) | 6% |
| N-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

7. Tabletten bzw. Boli

| | | |
|---|---|---|
| I | Wirkstoff aus Tabelle I | 33,0% |
| | Methylcellulose | 0,80% |
| | Kieselsäure hochdispers | 0,80% |
| | Maisstärke | 8,40% |
| II | Milchzucker krist. | 22,50% |
| | Maisstärke | 17,00% |
| | mikrokrist. Cellulose | 16,50% |
| | Magnesiumstearat | 1,00% |

I Methylcellulose in Wasser einrühren und quellen lassen; Kieselsäure in die Quellung einrühren und homogen suspendieren. Wirkstoff und Maisstärke mischen. In diese Mischung die wässrige Suspension einarbeiten und zu einem Teig kneten. Diese Masse durch ein Sieb 12 M granulieren und Trocknen.

II Alle 4 Hilfsstoffe gut mischen.

III Phasen I und II mischen und zu Tabletten oder Boli pressen.

Biologische Beispiele

Die anthelmintische Wirksamkeit wird anhand folgender Versuche demonstriert:

Beispiel 8 : Versuch an mit Nematoden wie Haemonchus concortus und Trichostrongylus colubriformis infizierten Schafen

Der Wirkstoff wird in Form einer Suspension mittels einer Magensonde oder durch Injektion in den Pansen Schafen verabreicht, die vorher mit Nematoden wie Haemonchus concortus und Trichostrongylus colubriformis künstlich infiziert wurden. Pro Versuch resp. pro Dosis werden 1 bis 3 Tiere verwendet. Jedes Schaf wird mit nur einer einzigen Dosis behandelt.

Eine erste Evaluierung erfolgt dadurch, dass die Anzahl der vor und nach der Behandlung im Kot der Schafe ausgeschiedenen Wurmeier verglichen werden.
Sieben bis zehn Tage nach der Behandlung werden die Schafe getötet und seziert. Die Auswertung erfolgt durch Auszählung der nach der Behandlung im Darm zurückbleibenden Würmer. Gleichzeitig und gleichartig infizierte aber unbehandelte Schafe dienen als Kontrolle resp. Vergleich.

Schafe, die mit einer Suspension eines Wirkstoffes aus der Tabelle I behandelt werden, zeigen im Vergleich zu unbehandelten, aber infizierten Vergleichsgruppen einen um 95 bis 100% reduzierten Nematodenbefall. Die Wirkungsdosis beträgt 10 mg/kg.

Beispiel 9 : Versuche an mit Cestoden wie Moniezia benedeni infizierten Schafen

Der Wirkstoff wird in Form einer Suspension mittels einer Magensonde oder durch Injektion in den Pansen Schafen verabreicht, die vorher mit Cestoden wie Moniezia benedeni infiziert wurden. Pro Versuch resp. pro Dosis werden 3 Tiere verwendet. Jedes Schaf wird mit nur einer einzigen Dosis behandelt.

Sieben bis zehn Tage nach der Behandlung werden die Schafe getötet und seziert. Die Auswertung erfolgt durch Auszählung der nach der Behandlung im Darm zurückbleibenden Würmer. Gleichzeitig und gleichartig infizierte aber unbehandelte Schafe dienen als Kontrolle resp. Vergleich. In diesem Versuch bewirken die Wirkstoffe Nr. 1-8 aus der Tabelle I eine vollständige Reduktion des Cestodenbefalls. Die Wirkungsdosis beträgt 15 mg/kg.

Beispiel 10: Versuch an mit Fasciola hepatica infizierten Schafen

Der Wirkstoff wird in Form einer Suspension mittels einer Magensonde oder durch Injektion in den Pansen Schafen verabreicht, die vorher mit Fasciola hepatica künstlich infiziert wurden. Pro Versuch resp. pro Dosis werden 3 Tiere verwendet. Jedes Tier wird mit nur einer einzigen Dosis behandelt.

Eine erste Evaluierung erfolgt dadurch, dass die Anzahl der vor und nach der Behandlung im Kot der Schafe ausgeschiedenen Wurmeier verglichen werden.

Drei bis vier Wochen nach der Behandlung werden die Schafe getötet und seziert. Die Auswertung erfolgt durch das Auszählen der nach der Behandlung in den Gallengängen zurückgebliebenen Leberegel. Gleichzeitig und gleichartig infizierte aber unbehandelte Schafe dienen als Kontrolle resp. Vergleich. Der Unterschied der in den beiden Gruppen festgestellten Anzahl von Leberegeln ergibt den Wirkungsgrad des geprüften Wirkstoffs.

In diesem Versuch zeigen die Wirkstoffe 1 und 3-8 aus der Tabelle I eine 95 bis 100%-ige Wirksamkeit gegen Fasciola hepatica. Die Wirkungsdosis beträgt 30 mg/kg.

Patentansprüche

1. Anthelmintisches Mittel, dadurch gekennzeichnet, dass es als aktive Komponente mindestens eine Verbindung der allgemeinen Formel I

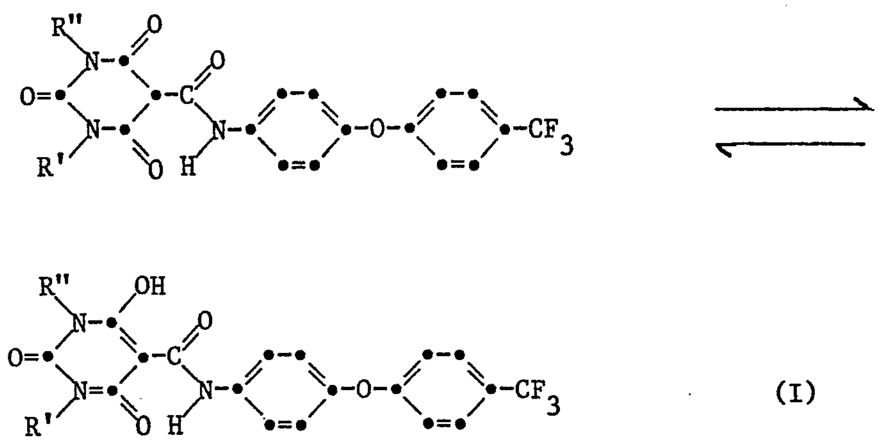

(I)

in welcher R' und R" unabhängig voneinander Wasserstoff oder $C_1$-$C_5$-Alkyl bedeuten, einschliesslich ihrer tautomeren Formen und ihrer Salze, neben physiologisch verträglichen Trägerstoffen und weiteren Formulierungshilfsmitteln enthält.

2. Mittel gemäss Anspruch 1, dadurch gekennzeichnet, dasd es einen Wirkstoff von 0,1 bis 99,0 Gew.-% und einen Gehalt an Trägerstoffen und weiteren Formulierungshilfsmitteln von 99,9 bis 1 Gew.-% besitzt.

3. Verfahren zur Bekämpfung parasitärer Helminthen, dadurch gekennzeichnet, dass man einem Tier eine anthelmintisch wirksame Menge einer Verbindung der Formel I, ein Tautomeres oder ein Salz davon gemäss Anspruch 1 verabreicht.

4. Verwendung einer Verbindung der Formel I, eines Tautomeren oder eines Salzes davon gemäss Anspruch 1 zur Bekämpfung von parasitären Helminthen.

5. Verwendung einer Verbindung der Formel I, eines Tautomeren oder eines Salzes davon gemäss Anspruch 1 zur Bekämpfung von Nematoden.

6. Verwendung einer Verbindung der Formel I, eines Tautomeren oder eines Salzes davon gemäss Anspruch 1 zur Bekämpfung von Cestoden.

7. Verwendung einer Verbindung der Formel I, eines Tautomeren oder eines Salzes davon gemäss Anspruch 1 zur Bekämpfung von Trematoden.

8. Verbindungen der Formel I

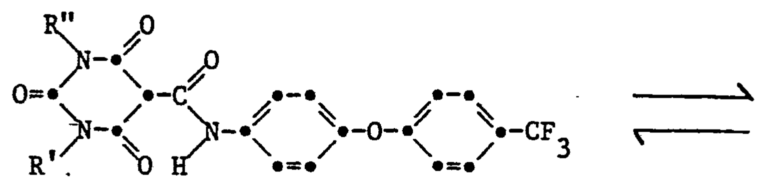

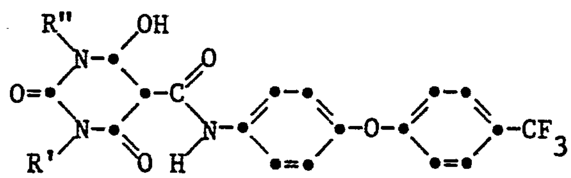

(I)

in welcher R' und R" unabhängig voneinander Wasserstoff oder $C_1$-$C_5$-Alkyl bedeuten, einschliesslich ihrer tautomeren Formen und ihrer Salze.